Europäisches Patentamt

European Patent Office

Office européen des brevets

(11) Numéro de publication: **0 516 507 B1**

(12) **FASCICULE DE BREVET EUROPEEN**

(45) Date de publication du fascicule du brevet: **06.12.95**  (51) Int. Cl.⁶: **C07C 15/00**

(21) Numéro de dépôt: **92401373.3**

(22) Date de dépôt: **20.05.92**

(54) **Procédé de production d'hydrocarbures liquides à partir du gaz naturel, en présence d'un catalyseur à base de zéolithe et de gallium.**

(30) Priorité: **21.05.91 FR 9106195**

(43) Date de publication de la demande:
**02.12.92 Bulletin 92/49**

(45) Mention de la délivrance du brevet:
**06.12.95 Bulletin 95/49**

(84) Etats contractants désignés:
**DE DK GB IT NL**

(56) Documents cités:
**WO-A-86/05176**
**FR-A- 2 629 451**

**PATENT ABSTRACTS OF JAPAN vol. 12, no. 181 (C-499)(3028) 27 Mai 1988 & JP-A-62 285 987**

(73) Titulaire: **INSTITUT FRANCAIS DU PETROLE**
**4, Avenue de Bois Préau**
**F-92502 Rueil-Malmaison (FR)**

(72) Inventeur: **Alario, Fabio**
**70 bis, rue Georges Clémenceau**
**F-94210 La Varenne (FR)**
Inventeur: **Cameron, Charles**
**118, rue d'Assas**
**F-75006 Paris (FR)**
Inventeur: **Joly, Jean-François**
**20, rue Béranger**
**F-75003 Paris (FR)**

## Description

La présente invention concerne un procédé de production d'hydrocarbures liquides à partir du gaz naturel. Plus spécifiquement cette invention concerne la transformation du gaz naturel, dont le constituant majeur est le méthane, en produits liquides plus facilement transportables. Encore plus spécifiquement, cette invention concerne un procédé caractérisé en ce que :

(a) on sépare le gaz naturel en, au moins deux fractions, une première fraction du gaz enrichie en méthane et une deuxième fraction enrichie en alcanes $C_2^+$ (éthane, propane et alcanes supérieurs),

(b) on oxyde sélectivement au moins une partie du méthane par de l'oxygène moléculaire en présence d'un catalyseur de couplage oxydant du méthane,

(c) on mélange, au moins en partie, la fraction enrichie en alcanes $C_2^+$ à l'effluent de l'étape d'oxydation sélective quand au moins 80 % de l'oxygène moléculaire introduit à l'étape (b) ont déjà été consommés dans l'étape (b),

(d) on pyrolyse le mélange résultant de l'étape (c),

(e) après avoir amené la température du mélange de l'étape (d) à une température comprise entre 300 °C et 750 °C et plus particulièrement entre 420 °C et 550 °C, on transforme, en partie au moins, les oléfines, et éventuellement en partie des $C_2^+$ alcanes, en aromatiques en présence d'un catalyseur particulier contenant une zéolithe, du gallium, un ou plusieurs métaux du groupe VIII et/ou du rhénium, un ou plusieurs métaux additionnels définis plus loin, éventuellement un métal alcalin ou alcalino-terreux et éventuellement une matrice; La composition du catalyseur est indiquée plus loin.

Les principes de base du procédé d'oxypyrolyse du gaz naturel, qui combine l'oxydation sélective du méthane et la pyrolyse des alcanes $C_2^+$ formés et ajoutés à l'effluent d'oxydation, sont décrits dans trois brevets français (2 629 451, 2 636 627 et 2 641 531) et dans un article (Appl. Catal., vol. 58 (1990) 269). Le document WO-A-8.605.176 décrit la combinaison d'un procédé de couplage oxydant et d'oligomérisation des oléfines formées.

Dans une première étape, étape (a), de ce procédé, le gaz naturel est séparé en au moins deux fractions, la première étant du méthane appauvri en alcanes $C_2^+$ (éthane, propane et alcanes supérieurs) et la deuxième fraction étant composée des alcanes supérieurs $C_2^+$ appauvris en méthane. Une partie au moins de la première fraction est ensuite mélangée avec de l'oxygène (pur ou enrichi) jusqu'à 0,4 mol d'oxygène moléculaire par mole de carbone. Le mélange peut contenir avantageusement des quantités importantes de vapeur d'eau. La quantité molaire de vapeur d'eau par rapport au méthane peut être entre 0 et 4, de préférence entre 0 et 2, de préférence encore entre 0 et 1 et plus particulièrement entre 0,05 et 0,50. La vapeur d'eau permet la manipulation des mélanges oxygène/hydrocarbures avec un degré de sécurité plus important et permet l'augmentation simultanée de la sélectivité en produits de couplage et de la conversion du méthane dans l'étape d'oxydation sélective du méthane.

La deuxième étape du procédé, étape (b), consiste à consommer l'oxygène moléculaire par une réaction d'oxydation catalytique sélective à des températures au moins supérieures à 650 °C, de préférence supérieures à 750 °C et de préférence encore supérieures à 800 °C. Généralement la pression est entre 1 et 15 bars, préférentiellement entre 1 et 10 bars et plus particulièrement entre 1 et 4 bars. Cette réaction, généralement appelée couplage oxydant du méthane, est effectuée en présence d'un catalyseur stable à haute température. Les catalyseurs stables à haute température sont généralement ceux qui contiennent au moins un oxyde réfractaire tel que la magnésie, l'oxyde de calcium, l'oxyde de strontium et les autres oxydes trouvés dans le tableau 3 de l'article paru dans Appl. Catal., vol. 67 (1990) 47. Les catalyseurs d'un intérêt particulier pour le couplage oxydant du méthane sont, entre autres, ceux décrits dans le brevet français (2 629 451), et dans les articles parus dans Appl. Catal., vol. 67 (1990) 47 et Chem. Soc. Rev., vol. 18 (1989) 251.

L'étape d'oxydation sélective peut être effectuée dans un réacteur à lit fixe, un réacteur à lit mobile ou un réacteur à lit transporté. L'utilisation d'un réacteur à lit fixe est particulièrement avantageuse dans le cas où le catalyseur n'a pas de bonnes propriétés de résistance mécanique et pour des conversions par passe du méthane relativement faibles, par exemple inférieures à 20 %. Les réacteurs à lit mobile, tels que ceux à lit bouillonnant ou à lit transporté, sont très intéressants à mettre en oeuvre quand la conversion par passe est, par exemple, supérieure à 20 %. La circulation du catalyseur permet un meilleur contrôle de la température par échange thermique entre la charge, le catalyseur et les effluents.

Quel que soit le réacteur utilisé pour l'étape d'oxydation sélective du méthane, cette étape d'oxydation sélective est fortement exothermique. Donc, il est très important d'abaisser la température de l'effluent pour limiter la formation d'acétylène et de coke qui peuvent être formés aux temps de contact élevés à température élevée. Pour cette raison il est souvent avantageux d'injecter, dans cet effluent chaud, au moins une partie de la deuxième fraction, composée d'alcanes supérieurs $C_2^+$ appauvris en méthane,

2

provenant de la première étape. Les hydrocarbures $C_2^+$, généralement paraffiniques, servent à abaisser la température en effectuant une trempe thermochimique, autrement dit : ils absorbent la chaleur dégagée par l'étape d'oxydation et sont transformés en oléfines et en hydrogène.

La troisième étape, étape (c), consiste à additionner au moins en partie les paraffines $C_2^+$, obtenues à l'étape (a), dans l'effluent d'oxydation sélective du méthane quand au moins 80 %, et de préférence au moins 95 %, de l'oxygène moléculaire introduit à l'étape (b) ont déjà été consommés dans la deuxième étape. Il y a trois avantages pour ce mode opératoire :

1. il n'est pas nécessaire d'opérer, dans l'étape (b) d'oxydation sélective du méthane, en présence des hydrocarbures $C_2^+$ plus oxydables que le méthane ;

2. l'addition des hydrocarbures $C_2^+$ de l'effluent d'oxydation sélective du methane, après qu'au moins 80 % et de préférence au moins 95 % de l'oxygène moléculaire introduit à l'étape (b) aient déjà été consommés, permet l'utilisation de la majorité de l'oxygène moléculaire pour l'activation du méthane et non pas pour la deshydrogénation des alcanes $C_2^+$ ;

3. l'addition des hydrocarbures $C_2^+$ dans l'effluent de l'oxydation sélective du méthane, après qu'au moins 80 % et de préférence au moins 95 % de l'oxygène moléculaire introduit à l'étape (b) aient déjà été consommés, permet la transformation par absorption de chaleur des alcanes $C_2^+$ en oléfines et en hydrogène, ce dernier pouvant être utilisé pour la récupération de carbone, par exemple en hydrogénant le CO en méthane.

La quatrième étape, étape (d), consiste à maintenir la température des effluents combinés pendant un temps suffisamment long pour permettre de préférence l'obtention d'un rapport molaire éthylène/éthane d'au moins 1,2 et de préférence supérieur à 1,4. Au cours de cette étape, le temps de séjour est compris généralement entre 50 millisecondes et 10 secondes, de préférence entre 100 millisecondes et 2 secondes.

Selon une méthode de production d'hydrocarbures liquides à partir du gaz naturel, qui ne fait pas partie de cette invention, les effluents gazeux sortant de la quatrième étape, étape (d), sont amenés à une température inférieure à 100°C, comprimés et introduits dans le système de séparation. Le gaz comprimé est ensuite débarrassé de l'eau et du $CO_2$ avant la réalisation d'une séparation des hydrocarbures supérieurs ($C_2$, $C_2$ =, $C_3$, $C_3$ = et hydrocarbures supérieurs) et des gaz légers (CO, $H_2$, $CH_4$). Les hydrocarbures supérieurs sont ensuite :

1. séparés pour produire des oléfines, (éthylène, propylène et oléfines supérieures) et des alcanes recyclables,

2. oligomérisés et/ou aromatisés pour l'obtention des hydrocarbures liquides et des alcanes recyclables, ou

3. dimérisés pour produire des oléfines $C_4^+$ liquides et des alcanes recyclables.

Selon la méthode de la présente invention, l'effluent provenant de l'oxypyrolyse, à l'état gazeux et contenant des gaz parmi lesquels des hydrocarbures tels que l'éthylène, l'éthane et le propylène, est avantageusement traité sur un catalyseur d'aromatisation. Il est en effet d'un grand intérêt de valoriser ces hydrocarbures $C_2$-$C_3$ en produits liquides, grands intermédiaires pétrochimiques par exemple.

Lesdits effluents de l'étape (d), qui sont ensuite utilisés pour la charge de l'étape (e), sont constitués :

1. d'au moins 40 % et au plus 95 % en poids de l'ensemble méthane plus eau,

2. d'au moins 5 %, de préférence au moins 10 %, et de préférence encore au moins 15 % en poids de composés hydrocarbures non-paraffiniques,

3. moins de 1 %, de préférence moins de 0,5 %, et de préférence encore moins de 0,1 % en poids d'oxygène moléculaire, et

4. d'autres composés (en quantités variables dépendant de la charge initiale de l'étape (a) et des conditions de fonctionnement des étapes (a) à (d)), tels que : $N_2$, CO, $CO_2$, $H_2$ et les alcanes $C_2^+$.

La cinquième étape, étape (e), consiste, donc, à amener les effluents de l'étape (d) à une température comprise entre 300°C et 750°C, et plus particulièrement entre 420°C et 550°C, puis l'effluent est mis en contact avec un catalyseur d'aromatisation particulier qui permet la transformation, en partie au moins, des oléfines en hydrocarbures liquides. La transformation de ces oléfines et éventuellement, en partie au moins, des alcanes $C_2^+$ en hydrocarbures liquides peut avoir des conséquences importantes sur les dimensions du système de séparation. De plus, le groupement d'une unité chaude, telle que l'unité d'aromatisation, avec d'autres unités chaudes avant les étapes froides de séparation, a des conséquences positives sur l'investissement nécessaire pour l'ensemble opérationnel de valorisation du gaz naturel.

L'invention possède deux avantages importants. La transformation au moins en partie des oléfines et éventuellement des alcanes $C_2^+$ en aromatiques avant d'entrer dans le système de séparation permet :

1. la réduction des dimensions du système de séparation, et

2. la réduction des utilités demandées grâce au regroupement d'une unité chaude (aromatisation) avec d'autres unités chaudes (oxydation, pyrolyse).

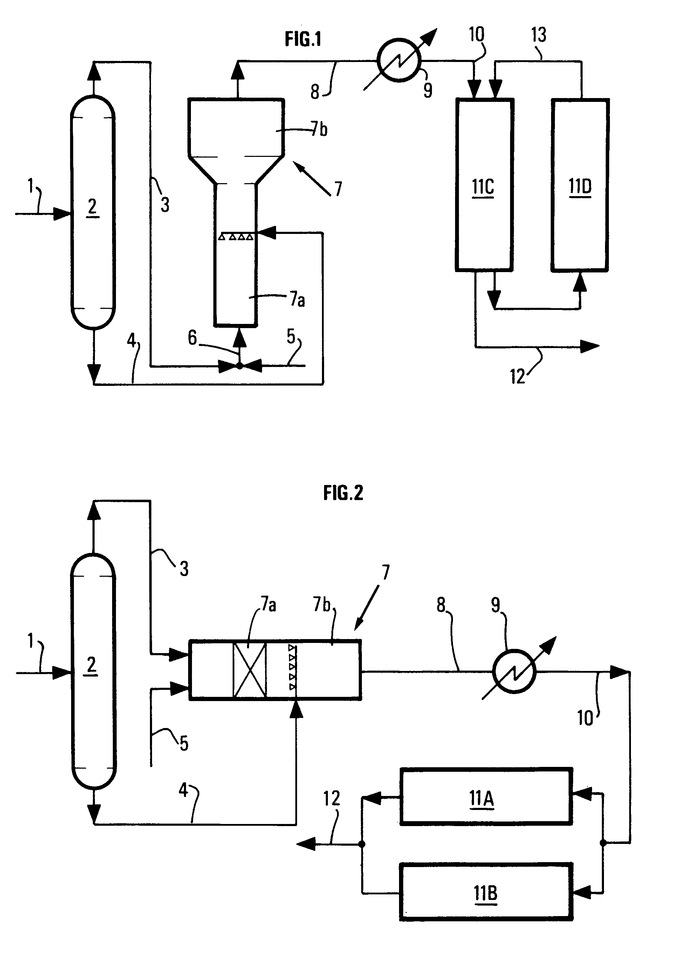

Les exemples suivants non limitatifs illustrent différents modes de réalisation qui peuvent être employés pour obtenir des hydrocarbures liquides à partir du gaz naturel selon l'invention. Ces exemples sont illustrés par les figures 1 et 2.

Une mise en oeuvre préférée de la présente invention consiste à utiliser un réacteur à lit bouillonnant pour les étapes d'oxydation sélective et de pyrolyse (oxypyrolyse) et un réacteur de type lit mobile pour l'aromatisation. Dans cette mise en oeuvre, le gaz enrichi en méthane (3) provenant de la séparation du gaz naturel (1) dans le séparateur (2) est mélangé à de l'oxygène moléculaire (5). Dans le cas où il est avantageux d'ajouter la vapeur d'eau dans la charge, celle-ci est généralement ajoutée au méthane ou à l'oxygène, ou au méthane et à l'oxygène avant de mélanger le méthane à l'oxygène. Il est très important de contrôler la température du mélange gazeux avant qu'il entre dans le réacteur. Pour ceci, on peut chauffer indépendamment les gaz contenant le méthane (3) et l'oxygène (5) ou l'ensemble des gaz (6). Les gaz préchauffés à une température généralement inférieure à 750°C et de préférence inférieure à 600°C, sont mis en contact avec le catalyseur de couplage oxydant du méthane dans la partie inférieure (7a) du réacteur (7) sur la figure 1 ou dans la partie en amont (7a) du réacteur (7) sur la figure 2.

Le catalyseur pour ce type de mise en oeuvre doit avoir une bonne résistance mécanique. La taille des particules est généralement entre 20 microns et 4 mm de diamètre. La taille des particules de catalyseur est variable en fonction des conditions opératoires de l'unité et en fonction de la densité du catalyseur. Bien qu'on ne puisse pas mentionner tous les catalyseurs du couplage oxydant du méthane potentiellement intéressants pour une application dans un réacteur à lit bouillonnant, on peut citer les catalyseurs décrits dans le brevet français (2 629 451), dans Appl. Catal., vol. 76 (1990) 47, dans Chem. Soc. Rev., vol. 18 (1989) 251, et par exemples des catalyseurs tels que : $BaCO_3/Al_2O_3$ (généralement, mais pas obligatoirement, en présence de quelques ppm d'une source de chlore dans la charge) ; $Pb/Al_2O_3$ (souvent en présence d'un métal alcalin et/ou un anion contenant du soufre ou du phosphore) ; des oxydes mixtes contenant Na, B, Mg et Mn (tels que $NaB_2Mg_4Mn_2O_x$); La/MgO (souvent en présence d'un ou plusieurs oxydes des métaux alcalino-terreux et/ou alcalins et/ou d'autres oxydes de lanthanides et éventuellement le bore) ; les combiniaisons Na ou K, un ou plusieurs oxydes des métaux alcalino-terreux et éventuellement le bore; des perovskites $MCeO_3$ (ou $M = Sr$ ou Ba) et les oxydes mixtes contenant du thorium ou du yttrium.

Après avoir subi l'oxydation sélective dans le réacteur (7) et à un endroit où au moins 80 % de l'oxygène moléculaire introduit à l'étape (b) ont été consommés, une partie au moins de la fraction du gaz enrichie en alcanes $C_2^+$ (4) est additionnée dans le réacteur. L'ajout de cette fraction peut être effectué dans le lit expansé du catalyseur (figure 1) ou à un niveau supérieur au catalyseur (telle que dans la zone de désengagement).

Après un temps de résidence suffisamment long, dans le réacteur (étape (d) du procédé), pour obtenir le taux d'oléfines désiré, les gaz sont conduits par la ligne (8) à un échangeur (9) où la température de ces gaz est abaissée à une température comprise entre 300°C et 750°C, et plus particulièrement entre 420°C et 550°C. Lesdits effluents sont ensuite envoyés sur la figure 1 par la ligne (10) vers le réacteur d'aromatisation (11c). Si la prression de fonctionnement du réacteur d'aromatisation (11) est supérieure à la pression dans la ligne (10), lesdits effluents peuvent être avantageusement comprimés à ce moment.

Dans la figure 1, le réacteur d'aromatisation (11c), dans une des mises en oeuvre préférées, reçoit la charge à aromatiser avec du catalyseur frais par la partie supérieure du réacteur. De cette manière, le catalyseur désactivé par cokage est enlevé en bas du réacteur. Le catalyseur désactivé subit ensuite une régénération en (11d) avant d'être chargé à nouveau par la ligne (13) dans la partie supérieure du réacteur. La charge, entrant dans le réacteur, est mise en contact avec un catalyseur d'aromatisation qui, dans la présente invention, est défini comme ci-dessous :

Le catalyseur contiendra :

1/ une zéolithe,

2/ du gallium,

3/ au moins un métal choisi dans le groupe constitué par les métaux du groupe VIII et le rhénium,

4/ au moins un métal additionnel,

5/ éventuellement au moins un métal alcalin ou alcalino-terreux et

6/ éventuellement au moins une matrice.

Le catalyseur composite contient de préférence une zéolithe MFI sous forme hydrogène, éventuellement du gallium dans sa charpente cristallisée, du gallium sous forme oxyde et une matrice généralement amorphe sur laquelle est déposé au moins un métal du groupe VIII, de préférence un métal de la famille du platine : Ru, Rh, Pd, Os, Ir et Pt et/ou du rhénium, au moins un métal additionnel choisi dans le groupe constitué par l'étain, le germanium, le plomb, l'indium, le gallium et le thallium, ou bien parmi les métaux tels que le cuivre, l'or, l'argent, le nickel, le fer, ou encore parmi les métaux du groupe VI tels que le chrome, le molybdène ou le tungstène, ladite matrice contenant éventuellement au moins un alcalin ou un

4

alcalino-terreux (de préférence le lithium ou le potassium).

La zéolithe MFI contenue dans le catalyseur de la présente invention peut être préparée par toutes les techniques décrites dans l'art antérieur. Ainsi la synthèse de ladite zéolithe peut être réalisée en milieu classique OH⁻ en présence ou en absence d'agent organique et/ou d'alcool. Le document "Synthesis of High Silica Zéolithes, P. Jacobs et J. Martens, Studies in Surface Science and Catalysis, vol. 33, (1987)" décrit la synthèse classique de la zéolithe MFI. La zéolithe MFI utilisée dans la présente invention peut également avoir été synthétisée dans des milieux moins classiques comme par exemple le milieu fluorure en présence (brevet EP-A-172068) ou en absence (demande de brevet français 90/16529) de composé organique. La zéolithe utilisée dans la présente invention contient les éléments silicium, aluminium et/ou gallium dans sa charpente cristallisée.

Après l'étape de synthèse, la zéolithe MFI est transformée en une forme hydrogène, notée H-MFI, par élimination pratiquement totale des composés organiques et/ou des cations alcalins ou alcalino-terreux qu'elle contient éventuellement après synthèse. Toutes les techniques décrites dans l'art antérieur peuvent être utilisées pour le passage à la forme hydrogène, comme par exemple les échanges ioniques suivis ou non de calcination, les traitements chimiques divers.

Toutes les zéolithes synthétisées dans l'un des systèmes suivants : Si-Al, Si-Al-Ga, Si-Ga, conviennent pour la présente invention. Cependant, leur rapport Si/T, où T représente Al et/ou Ga, est généralement supérieur à 7, de préférence supérieur à 25 et de manière encore plus préférée compris entre 40 et 500.

La zéolithe H-MFI, utilisée dans la présente invention, peut être soit soumise telle quelle à un dépôt de gallium soit mélangée avec les autres constituants du catalyseur, le gallium étant alors introduit ultérieurement dans ledit mélange.

De nombreuses techniques de dépôt de gallium peuvent être utilisées dans la présente invention parmi lesquelles on peut citer :

- les échanges ioniques grâce à l'utilisation de sels de gallium en solution aqueuse, par exemple du nitrate de gallium, du chlorure de gallium, de l'hydroxyde gallium,
- les imprégnations par des solutions desdits sels de gallium.

La teneur en gallium déposé sur le catalyseur composite est comprise entre 0,01 et 10 % en poids, de préférence entre 0,03 et 7 % en poids.

La matrice comprend au moins un oxyde réfractaire, et en particulier au moins un oxyde d'un métal choisi dans le groupe constitué par le magnésium, l'aluminium, le titane, le zirconium, le thorium, le silicium et le bore. De plus, elle peut aussi comprendre du charbon.

La matrice préférée est l'alumine, dont la surface spécifique peut être avantageusement comprise entre 10 et 600 m²/g et de préférence entre 150 et 400 m²/g.

Le catalyseur composite de la présente invention peut être préparé suivant deux voies dont le principe est donné ci-après, la réalisation pratique étant connue de l'homme de l'art.

**Première voie**

La zéolithe H-MFI est mélangée avec la matrice. Ce mélange peut être réalisé entre deux poudres, entre deux solides mis préalablement en forme séparément, entre une poudre et un solide mis préalablement en forme séparément. On peut également mettre en forme conjointement les deux solides par toutes les techniques décrites dans l'art antérieur : pastillage, extrusion, dragéfication, coagulation en goutte, séchage par atomisation. Lors de ces opérations de mise en forme, on peut si nécessaire ajouter un additif de mise en forme, choisi dans le groupe constitué par la silice et l'alumine. Ainsi on a procédé au mélange de la zéolithe avec la matrice et/ou à leur mise en forme, séparément ou conjointement. Après mélange et/ou mise en forme, on procède au dépôt des métaux et du gallium sur l'ensemble constitué de la matrice et de la zéolithe, l'ordre de dépôt étant peu important. On considère alors que la majeure partie des métaux, c'est-à-dire de 30 à 100 % en poids et de préférence de 60 à 100 % en poids par rapport au catalyseur composite, se retrouve sur la matrice. Le gallium se retrouve indifféremment sur la matrice ou au plus près des sites acides de la zéolithe H-MFI.

**Deuxième voie**

On dépose préalablement les métaux sur la matrice d'une part, et le gallium sur la zéolithe H-MFI d'autre part. Puis on procède au mélange de la zéolithe H-MFI contenant du gallium avec la matrice contenant des métaux et à leur mise en forme, séparément ou conjointement, à la mise en forme étant obtenue dans les mêmes conditions que précédemment. Dans une variante, la zéolithe, sur laquelle a été déposé le gallium, peut être mélangée à la matrice à l'une quelconque des étapes de dépôt des métaux sur la matrice.

La méthode préférée de préparation consiste à déposer le gallium sur la zéolithe, à déposer les métaux sur la matrice, puis ensuite à introduire la zéolithe chargée en gallium dans la matrice chargée en métaux par mise en forme des deux poudres. La mise en forme est de préférence effectuée après un broyage micronique, qui peut être réalisé en utilisant la technique du broyage humide.

Le catalyseur composite contient entre 1 et 99 % en poids de zéolithe, le complément à 100 % étant constitué par la matrice, les métaux et le gallium. La proportion respective de zéolithe et de matrice varie dans une large gamme, car elle dépend d'une part du rapport Si/T, où T est Al et/ou Ga, de la zéolithe et d'autre part de la teneur en métaux de la matrice dans le cas de la méthode préférée de préparation.

La matrice contenant les métaux, dans le cas de la méthode préférée de préparation, est généralement préparée suivant une procédure décrite ci-dessous.

On utilise pour l'imprégnation des métaux, soit une solution commune des métaux que l'on désire introduire, soit des solutions distinctes pour le ou les métaux du groupe VIII et/ou du rhénium et pour le ou les métaux additionnels choisi parmi l'étain, le plomb, l'indium, le germanium et le thallium et éventuellement l'élément choisi dans le groupe constitué par les métaux alcalins et les alcalino-terreux. Quand on utilise plusieurs solutions, on peut procéder à des séchages et/ou à des calcinations intermédiaires. On termine habituellement par une calcination, par exemple entre 500 et 1000°C, de préférence en présence d'oxygène moléculaire, par exemple en effectuant un balayage d'air.

Le ou les métaux du groupe VIII et/ou du rhénium peuvent être incorporés dans la matrice par imprégnation de ladite matrice à l'aide d'une solution, aqueuse ou non, contenant un sel ou un composé du groupe VIII et/ou du rhénium. Le platine est généralement introduit dans la matrice sous forme d'acide chloroplatinique, mais pour tout métal noble peuvent être également utilisés des composés ammoniaqués ou des composés tels que, par exemple, le chloroplatinate d'ammonium, le dichlorure de platine dicarbonyle, l'acide hexahydroxyplatinique, le chlorure de palladium, le nitrate de palladium.

Le métal additionnel choisi dans le groupe constitué par l'étain, le germanium, le plomb, l'indium, le gallium et le thallium, ou bien parmi les métaux tels que le cuivre, l'or, l'argent, le nickel, le fer ou encore parmi les métaux du groupe VI tels que le chrome, le molybdène ou le tungstène peut être introduit par l'intermédiaire de composés tels que par exemple les chlorures, les bromures et les nitrates d'étain, les halogénures, le nitrate, l'acétate et le carbonate de plomb, le chlorure et l'oxalate de germanium, le nitrate et le chlorure d'indium.

L'élément choisi dans le groupe constitué par les alcalins et les alcalino-terreux, de préférence le lithium ou le potassium, peut être introduit par l'intermédiaire de composés tels que par exemple l'halogénure, le nitrate, le carbonate, le cyanure et l'oxalate dudit élément.

L'emploi d'au moins un métal du groupe VIII ou du rhénium peut à titre d'exemple se faire grâce à l'utilisation de composés ammoniaqués.

Dans le cas du platine, on peut citer par exemple les sels de platine IV hexamines de formule $Pt(NH_3)_6X_4$ ; halogénopentamines de formule $(PtX(NH_3)_5)X_3$ ; tétrahalogénodiamines de formule $PtX_4(NH_3)_2$ ; les complexes de platine avec les halogènes-polycétones et les composés halogénés de formule $H(Pt(acac)_2X)$ ; X étant un halogène choisi dans le groupe formé par le chlore, le fluor, le brome et l'iode, et de référence X étant de chlore, et acac représentant le reste de formule $C_5H_7O_2$ dérivé de l'acétylacétone.

L'introduction du métal du groupe VIII ou du rhénium est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Après introduction du métal du groupe VIII ou du rhénium, le produit obtenu est éventuellement séché puis il est calciné de préférence à une température comprise entre 400 et 1000°C.

Après cette calcination, on peut procéder à l'introduction d'au moins un métal additionnel, précédée éventuellement d'une réduction à l'hydrogène à haute température, par exemple entre 300 et 500°C. Le métal additionnel M peut être introduit sous la forme d'au moins un composé organique choisi dans le groupe constitué par les complexes dudit métal, en particulier les complexes polycétoniques du métal M et les hydrocarbylmétaux tels que les alkyles, les cycloalkyles, les aryles, les alkylaryles et les arylalkyles métaux.

L'introduction du métal M est avantageusement effectuée à l'aide d'une solution du composé organométallique dudit métal dans un solvant organique. On peut également employer des composés organohalogénés du métal M. Comme composés du métal M, on peut citer en particulier le tétrabutylétain dans le cas où M est l'étain, le tétraéthylplomb dans le cas où M est le plomb et le triphénylindium dans le cas où M est l'indium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

Dans le cas où l'on n'utilise par le procédé de préparation tel que décrit ci-dessus, on peut aussi envisager d'introduire au moins un métal additionnel M avant l'introduction d'au moins un métal du groupe VIII ou du rhénium. Si le métal M est introduit avant le métal du groupe VIII ou du rhénium, le composé du métal M utilisé est généralement choisi dans le groupe constitué par l'halogénure, le nitrate l'acétate, le tartrate, le carbonate et l'oxalate du métal M. L'introduction est alors avantageusement effectuée en solution aqueuse. Dans ce cas, avant de procéder à l'introduction d'au moins un métal noble, on procède à une calcination sous air à une température comprise entre 400 et 1000°C.

Le catalyseur composite contient :

1. En poids par rapport à la matrice :
   - de 0,01 à 2 % et de préférence de 0,1 à 0,5 % d'au moins un métal du groupe VIII ou du rhénium,
   - de 0,005 à 2 % d'étain et de préférence de 0,01 à 0,5 % d'étain ou de 0,005 à 0,7 % et de préférence de 0,01 à 0,6 % d'au moins un métal choisi dans le groupe constitué par le germanium, l'indium, le plomb, le gallium et le thallium, ou bien parmi les métaux tels que le cuivre, l'or, l'argent, le nickel, le fer ou encore parmi les métaux du groupe VI tels que le chrome, le molybdène ou le tungstène, la teneur globale en métaux choisis dans le groupe constitué par l'étain, le germaniumn, l'indium et le plomb étant comprise entre 0,02 et 1,20 % et de préférence de 0,02 à 1,0 % et de manière encore plus préférée de 0,03 à 0,80 %,
   - éventuellement de 0,01 à 4 % et de préférence de 0,1 à 0,6 % d'au moins un métal choisi dans le groupe constitué par les alcalins et les alcalino-terreux, et de préférence choisi dans le groupe constitué par le lithium et le potassium.
2. entre 1 et 99 % en poids de zéolithe MFI forme hydrogène,
3. entre 0,01 et 10 % en poids, de préférence entre 0,03 et 7 % en poids de gallium.

A l'issue de la préparation, le catalyseur mis en forme contient une zéolithe H-MFI, du gallium, des métaux et une matrice, et on procède à une calcination sous air à une température comprise entre 450 et 1000°C. Le catalyseur ainsi calciné peut avantageusement subir un traitement d'activation sous hydrogène à haute temperature, par exemple comprise entre 300 et 500°C, afin d'obtenir une phase métallique plus active et au moins une partie de la phase oxyde de gallium de plus grande dispersion et localisée au plus proche des sites acides de la zéolithe H-MFI. La procédure de traitement sous hydrogène consiste par exemple en une montée lente de la température sous courant d'hydrogène jusqu'à la température maximale de réduction, comprise généralement entre 300 et 500°C et de préférence entre 350 et 450°C, suivie d'un maintien à cette température pour une durée comprise en général entre 1 et 6 heures.

Le catalyseur, obtenu par les procédures précédentes et pouvant éventuellement subir un traitement de calcination sous air à une température généralement comprise entre 350°C et 690°C, est mis en oeuvre pour la réaction d'aromatisation des gaz d'oxypyrolyse. Cette réaction revêt un intérêt particulier car elle permet de valoriser des hydrocarbures gazeux en des produits liquides à plus haute valeur ajoutée (benzène, toluène, xylènes principalement).

La charge, effluent d'oxypyrolyse et contenant, parmi d'autres composés, de l'éthylène et/ou de l'éthane et/ou du propylène, est mise en contact avec le catalyseur d'aromatisation ci-dessus décrit à une température comprise entre 300°C et 750°C, et plus particulièrement entre 420°C et 550°C, et avec un débit massique horaire de charge par rapport au poids de catalyseur (PPH) compris entre 0,5 et 150 h$^{-1}$, de préférence entre 1 et 80 h$^{-1}$. La pression de fonctionnement sera avantageusement comprise entre 1 et 18 bars, et de préférence entre 1 et 12 bars.

L'effluent du dit réacteur d'aromatisation (11c), enrichi en produits aromatiques, est transféré par le conduit (12) vers le système de séparation.

Une autre mise en oeuvre préférée de la présente invention consiste à utiliser un réacteur à lit fixe (7) pour les étapes d'oxydation sélective et de pyrolyse, de préférence mais pas nécessairement dans la même enceinte (donc ici 2 zones distinctes 7a et 7b). Selon une technique particulière (cf figure 2), l'aromatisation peut être effectuée dans au moins un réacteur décrit par (11a) et (11b), pouvant être mis "hors circuit" pour régénérer le catalyseur qu'il contient ("swing reactor" en langue anglaise). Dans cette mise en oeuvre de la présente invention, les catalyseurs utilisés pour l'oxydation sélective du méthane et l'aromatisation en partie au moins des oléfines, et éventuellement en partie des alcanes $C_2^+$, ne doivent pas nécessairement être très résistants à l'attrition. Il convient, donc, que les catalyseurs soient utilisés sous forme d'extrudés, de concassés ou de grains. Tous les catalyseurs précédemment cités pour une application dans un réacteur à lit bouillonnant peuvent être utilisés pour une application dans un réacteur à lit fixe. Le catalyseur pour ce type de mise en oeuvre ne nécessite pas une résistance mécanique particulièrement élevée.

Dans le cas où il est avantageux d'ajouter la vapeur d'eau dans la charge, celle-ci est généralement ajoutée au méthane ou à l'oxygène, ou au méthane et à l'oxygène avant de mélanger le méthane à l'oxygène. Il est très important de contrôler la température du mélange gazeux avant qu'il entre dans le réacteur. Pour ceci, dans le cas d'un réacteur à lit fixe, on chauffe indépendamment les gaz contenant le méthane (3) et l'oxygène (5) avant que le méthane soit mélangé à l'oxygène. Le mélange entre le méthane l'oxygène est effectué dans l'enceinte du réacteur (7) avant contact avec le catalyseur. Le mélange étant effectué dans le réacteur, le conduit (6) n'est éventuellement pas nécessaire pour cette mise en oeuvre. Néanmoins, il est souvent avantageux qu'une partie du préchauffage des gaz soit effectuée dans le réacteur. L'eau présente dans la charge est capable de recevoir, par rayonnement thermique, des calories dégagées par le catalyseur. Donc, par cette méthode une partie du préchauffage peut être effectuée dans le réacteur d'oxydation. Les gaz, préchauffés à une température généralement inférieure à 750°C et de préférence inférieure à 600°C, sont ensuite mis en contact avec le catalyseur du couplage oxydant du méthane dans le réacteur (7).

Après avoir subi l'oxydation sélective dans le réacteur (7) et à un endroit où au moins 80 % de l'oxygène moléculaire introduit à l'étape (b) ont été consommés, au moins une partie de la fraction du gaz enrichie en alcanes $C_2^+$ (4) est ajoutée dans le réacteur. L'endroit d'ajout de cette fraction se situe généralement après le lit catalytique pour raison de simplicité d'opération.

Après un temps de résidence suffisamment long (pyrolyse) pour obtenir le taux d'oléfines désiré (rapport molaire éthylène/éthane d'au moins 1,2), le gaz est acheminé vers le réacteur d'aromatisation par les lignes (8) et (10) au travers de l'unité (9). L'ensemble du réacteur d'aromatisation, décrit par (11a) et (11b), fonctionne en discontinu. La partie (11a) de l'ensemble du réacteur, par exemple, reçoit le gaz à aromatiser à une température et pendant un temps suffisamment long pour transformer une partie au moins des oléfines, et éventuellement une partie des alcanes $C_2^+$, en aromatiques. Pendant une période, au moins, de cette transformation dans la partie (11a) de l'ensemble de réacteur, la partie (11b) est mise en régénération pour enlever le carbone qui s'est déposé sur le catalyseur durant le cycle précédent. Après cette étape de régénération, la partie (11b) est remise en service et ainsi de suite.

L'éffluent aromatisé dans le réacteur d'aromatisation est ensuite envoyé vers le système de séparation par la ligne (12).

Les exemples qui suivent précisent le procédé sans toutefois en limiter la portée.

EXEMPLE 1 : Préparation de l'alumine renfermant du platine, de l'étain et du lithium (catalyseur A)

On ajoute à 100 g de support d'alumine, 100 cm³ d'une solution aqueuse de nitrate de lithium, on laisse en contact 6 heures, on essore, on sèche en 1 heure à 100-120°C puis on calcine sous courant d'air sec 2 heures à 350°C.

Sur le produit calciné contenant le lithium, on procède à l'imprégnation du platine en ajoutant au solide 100 cm³ d'une solution d'acétylacétonate de platine dans le toluène. La concentration en platine de cette solution est égale à 3 g/l. On laisse 6 heures en contact, on sèche 1 heure à 100-120°C puis on calcine 2 heures à 530°C. Puis on réduit sous courant d'hydrogène sec pendant 2 heures à 450°C.

Après réduction, le produit contenant le lithium et le platine est immergé dans le n-heptane à raison de 100 g de solide pour 300 cm³ de solvant hydrocarboné. Ensuite, on injecte dans le n-heptane contenant le catalyseur, 3 g de solution de tétra-n-butylétain dans le n-heptane (à 10 % poids d'étain). Le contact entre le solide contenant le platine et la solution de tétra-n-butylétain est maintenu pendant 6 heures à la température de reflux de l'heptane. La solution d'imprégnation est alors évacuée et l'on procède à 3 lavages par du n-heptane pur à la température de reflux du n-heptane. Le catalyseur est ensuite séché. Il subit alors une calcination sous air pendant 2 heures à 500°C suivie d'une réduction sous courant d'hydrogène à 450°C avant d'être chargé dans le réacteur.

L'alumine renferme alors, en poids, 0,3 % de platine, en poids, 0,3 % d'étain et 0,5 % de lithium.

EXEMPLE 2 : Zéolithe MFI forme hydrogène et catalyseur B contenant cette zéolithe H-MFI et du gallium.

On utilise une zéolithe H-MFI forme hydrogène fournie par la société CON-TEKA sous la référence CBV 5020. Cette zéolithe H-MFI est caractérisée par un rapport Si/Al égal à 29, une teneur en sodium égale à 0,014 % en poids et un volume poreux mesuré par adsorption d'azote à 77K de 0,192 cm³/g.

Le gallium est déposé sur cette zéolithe par échange ionique. La solution d'échange est préparée à partir de nitrate de gallium $Ga(NO_3)_3$ de normalité 0,15 N. Le pH de la solution de nitrate de gallium est ajusté à la valeur de 2 par ajout d'ammoniaque.

La teneur en gallium atteinte, après trois échanges ioniques successifs de la zéolithe H-MFI avec la solution décrite ci-dessus, dans le catalyseur B ainsi obtenu est égale à 3,5 % en poids.

EXEMPLE 3 : Transformation d'un effluent d'oxypyrolyse

Le catalyseur C, obtenu après pastillage d'un mélange équi-massique du catalyseur A de l'exemple 1 et du catalyseur B de l'exemple 2, a été mis en oeuvre pour l'aromatisation d'un effluent d'oxypyrolyse, dont la composition pondérale est indiquée dans le tableau 1. Dans ce qui suit, l'effluent d'oxypyrolyse (10) est dénommé "charge", et ses produits de transformation sur le catalyseur d'aromatisation sont dénommés "produits".

TABLEAU 1

|  | Charge % en poids |
|---|---|
| $C_1$ | 55,7 |
| $C_2^=$ | 15,0 |
| $C_2$ | 2,51 |
| $C_3^=$ | 0,96 |
| $H_2$ | 1,30 |
| $CO$ | 0,93 |
| $CO_2$ | 8,40 |
| $H_2O$ | 15,2 |

Le catalyseur d'aromatisation a été chargé dans un réacteur à lit fixe (11) ou les conditions opératoires étaient les suivantes :

- Température : 500 °C,
- Pression : atmosphérique,
- Débit horaire de charge liquide égal à 30 fois le poids du catalyseur.

Les résultats, en termes de pourcentages massiques des produits trouvés dans la ligne (12), sont présentés dans le tableau 2.

TABLEAU 2

|  | Produits % en poids |
|---|---|
| $C_1$ | 56,4 |
| $C_2^=$ | 2,02 |
| $C_2$ | 2,98 |
| $C_3^=$ | 0,70 |
| $C_3$ | 1,01 |
| $C_4^=$ | 0,70 |
| $C_4$ | 0,79 |
| $C_5 +$ non aromatiques | 0,35 |
| Aromatiques | 9,11 |
| $H_2$ | 1,41 |
| $CO$ | 1,02 |
| $CO_2$ | 8,25 |
| $H_2O$ | 15,3 |

**Revendications**

1. Procédé de production d'hydrocarbures liquides à partir du gaz naturel caractérisé en ce que :

(a) on sépare le gaz naturel en, au moins deux fractions, une première fraction du gaz enrichie en méthane et une deuxième fraction enrichie en alcanes $C_2^+$ (éthane, propane et alcanes supérieurs),

(b) on oxyde sélectivement au moins une partie du méthane par de l'oxygène moléculaire en présence d'un catalyseur de couplage oxydant du méthane,

(c) on mélange, au moins en partie, la fraction enrichie en alcanes $C_2^+$ à l'effluent de l'étape d'oxydation sélective quand au moins 80 % de l'oxygène moléculaire introduit à l'étape (b) ont déjà été consommés dans l'étape (b),

(d) on pyrolyse le mélange résultant de l'étape (c),

(e) après avoir amené la température du mélange de l'étape (d) à une température comprise entre 300°C et 750°C, on transforme, en partie au moins, les oléfines, et éventuellement en partie des alcanes $C_2^+$, en aromatiques en présence d'un catalyseur d'aromatisation qui renferme :

(●) une zéolithe,

(●) du gallium,

(●) au moins un métal choisi dans le groupe constitué par les métaux du groupe VIII de la famille du platine, et le rhénium,

(●) au moins un métal additionnel choisi dans le groupe constitué par le germanium, l'indium, le plomb, le gallium, le thallium ou bien parmi les métaux: du groupe VI tels que le chrome, le molybdène et le tungstène, ou bien parmi les métaux tels que le cuivre, l'or, l'argent, le nickel et le fer.

2. Procédé selon la revendication 1 dans lequel, au cours de l'étape (e), la température du mélange de l'étape (d) est amené à une température comprise entre 420°C et 550°C.

3. Procédé selon l'une des revendications 1 et 2 dans lequel, à l'issue de l'étape (a), une partie au moins de la première fraction (fraction méthane) est mélangée avec de l'oxygène dont la teneur peut atteindre 0,4 mol d'oxygène moléculaire par mole de carbone.

4. Procédé selon la revendication 3 dans lequel le mélange renferme une quantité molaire de vapeur d'eau comprise entre 0 et 4 par rapport au méthane.

5. Procédé selon l'une des revendications 1 à 4 dans lequel au moins une partie de la deuxième fraction ($C_2^+$) obtenue à l'étape (a) est injectée dans la zone d'oxydation sélective du méthane (étape (b)).

6. Procédé selon l'une des revendications 1 à 5 dans lequel, au cours de l'étape (d), on maintient la température des effluents combinés obtenus à l'étape (c), jusqu'à l'obtention par pyrolyse d'un rapport molaire éthylène/éthane d'au moins 1,2, ladite température étant comprise entre 800°C et 950°C, avec un temps de séjour compris entre 50 millisecondes et 10 secondes.

7. Procédé selon l'une des revendications 1 à 6 dans lequel le catalyseur d'aromatisation renferme en outre au moins un métal alcalin ou alcalino-terreux.

8. Procédé selon l'une des revendications 1 à 7 dans lequel le catalyseur d'aromatisation renferme en outre une matrice.

9. Procédé selon l'une des revendications 1 à 8 dans lequel la zéolithe est une MFI.

10. Procédé selon la revendication 8 dans lequel le catalyseur renferme 1 à 99 % en poids de zéolithe, 0,01 à 10 % en poids de gallium, le complément à 100 % étant constitué par le support (matrice) chargé en agents actifs (métaux).

11. Procédé selon la revendication 10 dans lequel la zéolithe est une MFI.

12. Procédé selon l'une des revendications 8 à 10 dans lequel le catalyseur d'aromatisation renferme, en poids, par rapport à la matrice :

(a) 0,01 à 2 % d'au moins un métal du groupe VIII et/ou du rhénium,
(b) 0,005 à 2 % de métal additionnel lorsque celui-ci est l'étain ou 0,005 à 0,7 % de métal additionnel lorsque ce dernier n'est pas l'étain,
(c) 0,01 à 4 % d'au moins un métal alcalin ou alcalino-terreux.

**Claims**

1. A method of producing liquid hydrocarbons from natural gas, characterised in that :
   (a) natural gas is separated into at least two fractions, a first fraction of gas enriched with methane and a second fraction enriched with C2+ alkanes (ethane, propane and higher alkanes)
   (b) at least part of the methane is oxidised selectively by molecular oxygen in the presence of a catalyst for oxidising linkage of methane
   (c) at least part of the fraction enriched with C2+ alkanes is mixed with the effluent from the selective oxidation stage, when at least 80% of the molecular oxygen introduced at stage (b) has been consumed at stage (b)
   (d) the mixture resulting from stage (c) is pyrolysed
   (e) when the temperature of the mixture from stage (d) has been brought to 300 to 750°C, at least part of the olefins and possibly part of the C2+ alkanes are converted to aromatics in the presence of an aromatising catalyst containing:
   * a zeolite
   * gallium
   * at least one metal selected from the group comprising Group VIII metals of the platinum family, and rhenium
   * at least one additional metal, selected in the group consisting of germanium, indium, lead, gallium, thallium, or of the group of metals of group VI such as chromium, molybdenum, tungsten, or of metals such as copper, gold, silver, nickel and iron.

2. The method of claim 1 wherein, during stage (e), the temperature of the mixture from stage (d) is brought to a temperature from 420 to 550°C.

3. The method of claim 1 or 2 wherein, at the end of stage (a), at least part of the first fraction (the methane fraction) is mixed with oxygen, the content of which may be up to 0.4 mol of molecular oxygen per mol of carbon.

4. The method of claim 3, wherein the mixture contains a molar quantity of water vapour of from 0 to 4 relative to the methane.

5. The method of any of claims 1 to 4, wherein at least part of the second fraction (C2+) obtained at stage (a) is injected into the zone for selective oxidation of methane (stage b).

6. The method of any of claims 1 to 5 wherein, during stage (d), the temperature of the combined effluents obtained at stage (c) is maintained until an ethylene/ethane molar ratio of at least 1.2:1 is obtained by pyrolysis, the temperature being from 800 to 950°C, with a dwell time from 50 milliseconds to 10 seconds.

7. The method of any of claims 1 to 6, wherein the aromatising catalyst further contains at least one alkali metal or alkaline earth metal.

8. The method of any of claims 1 to 7, wherein the aromatising catalyst further contains a matrix.

9. The method of any of claims 1 to 8, wherein the zeolite is an MFI.

10. The method of claim 8, wherein the catalyst contains 1 to 99% by weight of zeolite, 0.01 to 10% by weight of gallium, with the balance to 100% being formed by the carrier (matrix) charged with active agents (metals).

11. The method of claim 10, wherein the zeolite is an MFI.

**12.** The method of any of claims 8 to 10, wherein the aromatising catalyst contains, by weight relative to the matrix:

    (a) 0.01 to 2% of at least one Group VIII metal and/or rhenium

    (b) 0.005 to 2% of additional metal when this is tin, or 0.005 to 0.7% of additional metal when this is not tin

    (c) 0.01 to 4% of at least one alkali metal or alkaline earth metal.

**Patentansprüche**

**1.** Verfahren zur Herstellung von flüssigen Kohlenwasserstoffen aus Erdgas, dadurch gekennzeichnet, daß:

    (a) man das Erdgas in wenigstens zwei Fraktionen, eine erste mit Methan angereicherte Gasfraktion und eine zweite mit Alkanen $C_2^+$ (Ethan, Propan und höheren Alkane) angereicherte Fraktion, trennt,

    (b) man selektiv wenigstens einen Teil des Methans durch molekularen Sauerstoff in Anwesenheit eines Kupplungskatalysators, der Methan oxidiert, oxidiert,

    (c) man wenigstens teilweise die mit Alkanen $C_2^+$ angereicherte Fraktion mit dem Abgang des Schrittes zur selektiven Oxidation mischt, wenn schon wenigstens 80 % des in den Schritt (b) eingeführten molekularen Sauerstoffs in dem Schritt (b) verbraucht sind,

    (d) man die aus dem Schritt (c) resultierende Mischung pyrolysiert,

    (e) man, nachdem die Temperatur der Mischung des Schrittes (d) auf eine Temperatur einschließlich zwischen 300 °C und 750 °C gebracht ist, teilweise wenigstens die Olefine und gegebenenfalls teilweise die Alkane $C_2^+$ in Anwesenheit eines Katalysators zur Aromatisierung in Aromaten umwandelt, der enthält:

    (■) einen Zeolith,

    (■) Gallium,

    (■) Wenigstens ein Metall, das aus der Gruppe ausgewählt ist, welche durch die Metalle der Gruppe VIII der Familie von Platin und Rhenium gebildet ist,

    (■) wenigstens ein zusätzliches Metall, das aus der Gruppe ausgewählt, welche durch Germanium, Indium, Blei, Gallium, Thallium und ebenso unter den Metallen der Gruppe VI, wie Chrom, Molybdän und Wolfram, oder auch unter den Metallen, wie Kupfer, Gold, Silber, Nickel und Eisen, gebildet ist.

**2.** Verfahren nach Anspruch 1, bei welchem im Laufe des Schrittes (e) die Temperatur der Mischung des Schrittes (d) auf eine Temperatur einschließlich zwischen 420 °C und 550 °C gebracht wird.

**3.** Verfahren nach einem der Ansprüche 1 und 2, bei welchem am Ausgang des Schrittes (a) ein Teil wenigstens der ersten Fraktion (Methanfraktion) mit Sauerstoff gemischt wird, dessen Gehalt 0,4 Mol molekularen Sauerstoffs pro Mol Kohlenstoff erreichen kann.

**4.** Verfahren nach Anspruch 3, bei welchem die Mischung eine molare Menge an Wasserdampf einschließlich zwischen 0 und 4 in bezug auf Methan enthält.

**5.** Verfahren nach einem der Ansprüche 1 bis 4, bei welchem wenigstens ein Teil der zweiten Fraktion ($C_2^+$), die aus dem Schritt (a) erhalten wird, in die Zone zur selektiven Oxidation des Methans (Schritt (b)) injiziert wird.

**6.** Verfahren nach einem der Ansprüche 1 bis 5, bei welchem man im Laufe des Schrittes (d) die Temperatur der kombinierten Abgänge, die aus dem Schritt (c) erhalten werden, bis zum Erhalt eines Molverhältnisses Ethylen/Ethan von wenigstens 1, 2 durch Pyrolyse beibehält, wobei die Temperatur einschließlich zwischen 800 °C und 950 °C mit einer Verweilzeit einschließlich zwischen 50 Millisekunden und 10 Sekunden beträgt.

**7.** Verfahren nach einem der Ansprüche 1 bis 6, bei welchem der Katalysator zur Aromatisierung außerdem wenigstens ein Alkali- oder Erdalkalimetall enthält.

**8.** Verfahren nach einem der Ansprüche 1 bis 7, bei welchem der Katalysator zur Aromatisierung außerdem wenigstens eine Matrix enthält.

**9.** Verfahren nach einem der Ansprüche 1 bis 8, bei welchem der Zeolith ein MFI-Zeolith ist.

**10.** Verfahren nach Anspruch 8, bei welchem der Katalysator 1 bis 99 Gew.-% Zeolith, 0,01 bis 10 Gew.-% Gallium enthält, wobei die Ergänzung auf 100 % durch den Träger (Matrix), der mit aktiven Wirkstoffen (Metallen) beladen ist, gebildet wird.

**11.** Verfahren nach Anspruch 10, bei welchem der Zeolith ein MFI-Zeolith ist.

**12.** Verfahren nach einem der Ansprüche 8 bis 10, bei welchem der Katalysator zur Aromatisierung in Gewichten im Verhältnis zu der Matrix enthält:

(a) 0,01 bis 2 % wenigstens eines Metalls der Gruppe VIII und/oder Rhenium,

(b) 0,005 bis 2 % eines zusätzlichen Metalls, wenn dieses Zinn ist, oder 0,005 bis 0,07 % eines zusätzlichen Metalls, wenn dieses letztere nicht Zinn ist,

(c) 0,01 bis 4 % wenigstens eines Alkali- oder Erdalkalimetalls.

**FIG.1**

**FIG.2**